(19) Europäisches Patentamt

European Patent Office

Office européen des brevets

(11) **EP 1 372 694 B1**

(12) **EUROPEAN PATENT SPECIFICATION**

(45) Date of publication and mention
of the grant of the patent:
**28.06.2006 Bulletin 2006/26**

(51) Int Cl.:
***A61K 38/08*** (2006.01)   ***A61K 51/00*** (2006.01)
***A61P 35/00*** (2006.01)

(21) Application number: **02706813.9**

(22) Date of filing: **26.03.2002**

(86) International application number:
**PCT/FI2002/000252**

(87) International publication number:
**WO 2002/076491 (03.10.2002 Gazette 2002/40)**

(54) **LIPOSOME TARGETING OF MATRIX METALLOPROTEINASE INHIBITORS**

LIPOSOMEN-TARGETING VON MATRIX-METALLOPROTEINASE-HEMMERN

CIBLAGE DE LIPOSOMES AU MOYEN D'INHIBITEURS DE METALLOPROTEINASES MATRICIELLES

(84) Designated Contracting States:
**AT BE CH CY DE DK ES FI FR GB GR IE IT LI LU MC NL PT SE TR**
Designated Extension States:
**LT LV SI**

(30) Priority: **26.03.2001 FI 20010620**

(43) Date of publication of application:
**02.01.2004 Bulletin 2004/01**

(73) Proprietor: **CTT Cancer Targeting Technologies OY**
**00790 Helsinki (FI)**

(72) Inventors:
• **PENATE MEDINA, Oula**
**FIN-00510 Helsinki (FI)**

• **KOIVUNEN, Erkki**
**FIN-00980 Helsinki (FI)**
• **KINNUNEN, Paavo**
**FIN-02660 Espoo (FI)**

(74) Representative: **Karvinen, Leena Maria et al**
**Oy Jalo Ant-Wuorinen Ab**
**Iso Roobertinkatu 4-6 A**
**00120 Helsinki (FI)**

(56) References cited:
**WO-A1-99/47550**

• **OULA PENATE MEDINA ET AL.: 'Binding of novel peptide inhibitors of type IV collagenases to phospholipis membranes and use in liposome targeting to tumor cells in vitro' CANCER RESEARCH vol. 61, May 2001, pages 3978 - 3985, XP002954535**

**Description**

**Field of the Invention**

The present invention relates to targeted cancer therapy and concerns specifically the use of small matrix metalloproteinase inhibitors in improving targeting of liposomes to cancer cells, and in enhancing the uptake thereof to such cells.

The invention thus also concerns the use of a composition comprising liposomes and certain small matrix metalloproteinase inhibitory peptides for the manufacture of a medical composition to treat tumor cells of a patient.

**Background of the Invention**

Matrix metalloproteinases (MMPs) constitute a family of enzymes capable of degrading the basement membrane and extracellular matrix (ECM), thus contributing to tissue remodeling and cell migration (Koivunen et al., 1999; Shapiro, 1997). MMPs can be divided into subgroups, one of which is constituted by the type IV collagenases or gelatinases, MMP-2 and MMP-9. Expression of gelatinases in normal cells such as trophoblasts, osteoclasts, neutrophils, and macrophages is tightly regulated. Similarly to other MNIPs, gelatinases are secreted in an inactive form (pro MMP) and proteolytic cleavage is needed for their activation.

Elevated or unregulated expression of gelatinases and other MMPs can contribute to the pathogenesis of several diseases, including tumor angiogenesis and metastasis, rheumatoid arthritis, multiple sclerosis, and periodontitis. Compounds inactivating gelatinases may thus provide potential therapeutic means for cancer and inflammatory disorders (Sorsa et al., 1994; Lauhio et al., 1991). Although a number of MMP inhibitors have been described, specific inhibitors of gelatinases have not been available (Lauhio et al., 1991). Recently, we screened random phage peptide libraries with the aim to develop a selective inhibitor against this MMP subgroup. The most active peptide derived, abbreviated CTT, was found to selectively inhibit the activity of NLMP-2 and MMP-9 of the MMP family members studied (Koivunen et al., 1999). CTT also inhibited endothelial and tumor cell migration *in vitro,* as well as tumor progression *in vivo* in mouse models, indicating the importance of gelatinases in tumor invasion.

WO publication 99/47550 discloses matrix metalloproteinase inhibitors of formula CXXHWGFXXC. It is mentioned in the application that the small size of these MMP-targeting peptides can be utilized to carry drugs to tumors.

Experiments in mice bearing tumor xenografts showed that CTT-displaying phages were accumulated in the tumor vasculature after their intravenous injection into the recipient mice. Targeting of the phage to tumors was inhibited by the coadministration of CTT peptide (Koivunen et al., 1999). These results suggest that CTT, besides being a potent antitumor agent itself by blocking cancer cell migration and angiogenesis, may also be utilized for targeting of chemotherapeutics to tumors.

In chemotherapy, only a fraction of the drug reaches the cancer cells, whereas the rest of the drug may damage normal tissues. Adverse effects can be reduced by the administration of cancer drugs encapsulated in liposomes (Lasic et al., 1995). Improved liposome compositions have been described, so as to enhance their stability and also to prolong their lifetime in the circulation (Tardi et al., 1996). Both *in vitro* and *in vivo* studies on the development of liposomes targeted to cancer cells have been reported (Northfelt et al., 1996; Adlakha-Hutcheon et al., 1999). Enhanced selectivity can be obtained by derivatizing the liposomes with specific antibodies recognizing plasma membrane antigens of target cells, thus augmenting the uptake of liposomes by cells (Storm and Crommelin, 1998). As both MMP-2 (Toth et al., 1997) and MMP-9 (Brooks et al., 1996) are bound by specific cell surface receptors, these enzymes represent potential receptors for liposome targeting to invasive cells, such as tumor cells and angiogenic endothelial cells.

Furthermore, phospholipids conjugated to monomethoxy polyethylene glycol (PEG) have been widely used ever since 1984 when Sears coupled, via an amide linkage, carboxy-PEG and purified soy phosphatidyl ethanolamine (PE) (Sears, 1984). The addition of PEG onto the liposome surface attracts a water shell to surround the liposome. This shell prevents the adsorption of various plasma proteins (opsonins) to the liposome surface so that liposomes are not recognized and taken up by the reticulo-endothelial system.

**Summary of the Invention**

The present invention is based on the finding that certain MMP inhibitory peptides improve the targeting of liposomes to cancer cells and enhance the uptake thereof by such cells.

Consequently, the invention is directed to the use of peptide compounds comprising a structure selected from the group consisting of the cyclic peptide CLPGHWGFPSC (SEQ ID NO:7) and peptides having the linear motif of S(X)$_y$HWGFXXS (SEQ ID NO:4 or 5), wherein X is any amino acid residue and y is an integer of 2 or 3, for the preparation of a composition to improve targeting of liposomes to tumor cells.

A further object of the invention is the use of peptide compounds comprising a structure selected from the group consisting of the cyclic peptide CLPGHWGFPSC and peptides having the linear motif of $S(X)_y$HWGFXXS, wherein X is any amino acid residue and y is an integer of 2 or 3, for the preparation of a composition to enhance the uptake of liposomes to tumor cells.

Still another object of the invention is the use of a composition comprising liposomes carrying at least one chemotherapeutic agent and at least one peptide compound selected from the group consisting of the cyclic peptide CLPGHWGFPSC and peptides having the linear motif of $S(X)_y$HWGFXXS, wherein X is any amino acid residue and y is an integer of 2 or 3 for the manufacture of a medical composition to treat tumor cells of a patient.

In a further, preferred embodiment of the invention polyethylene glycol (PEG) is attached to the liposomes, preferably onto the liposome surface, before mixing the peptide compound with the liposomes.

A still further object of the invention is a diagnostic or imaging test kit for detecting a suspected tumor in a patient, wherein the test kit comprises liposomes, at least one peptide compound selected from the group consisting of the cyclic peptide CLPGHWGFPSC and peptides having the linear motif of $S(X)_y$HWGFXXS, wherein X is any amino acid residue and y is an integer of 2 or 3, and a detectable label.

According to the invention peptide compounds as described are used to target a label to a suspected tumor. A radioactive or magnetic label can be attached to the peptide, or inside a liposome, or on the surface of the liposome, which is targeted to the site of the tumor by the peptide. The measurement is carried out by gamma imaging or autoradiography.

Another object of the invention is a diagnostic or imaging composition, which comprises liposomes, at least one peptide compound selected from the group consisting of the cyclic peptide CLPGHWGFPSC and peptides having the linear motif of $S(X)_y$HWGFXXS, wherein X is any amino acid residue and y is an integer of 2 or 3, and a detectable label.

A suitable detectable label for the purposes of this invention is a radioactive label, a magnetic particle or a fluorescent labeL

The invention is hereinbelow described in more detail referring to the accompanied drawings.

## Brief description of the drawings

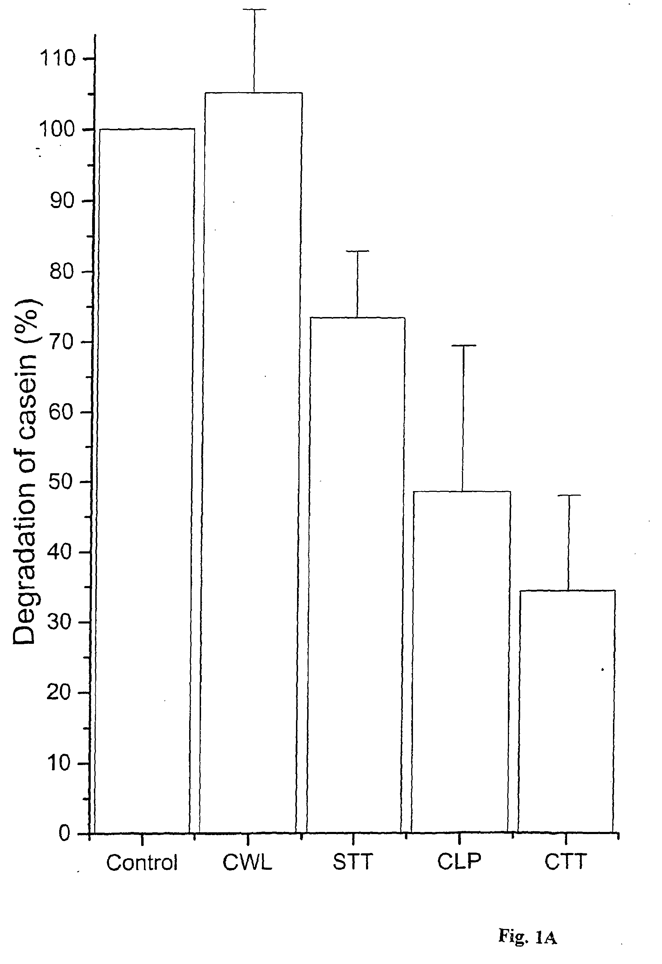

**Figure 1A.** Inhibition of MMP-2 activity by CTT, CLP, STT, and CWL peptides each at 85 µM concentration and assessed by casein zymography. Results are shown as the percentage of area digested, where uninhibited MMP-2 is taken as 100%. Error bars represent standard deviation for three separate experiments.

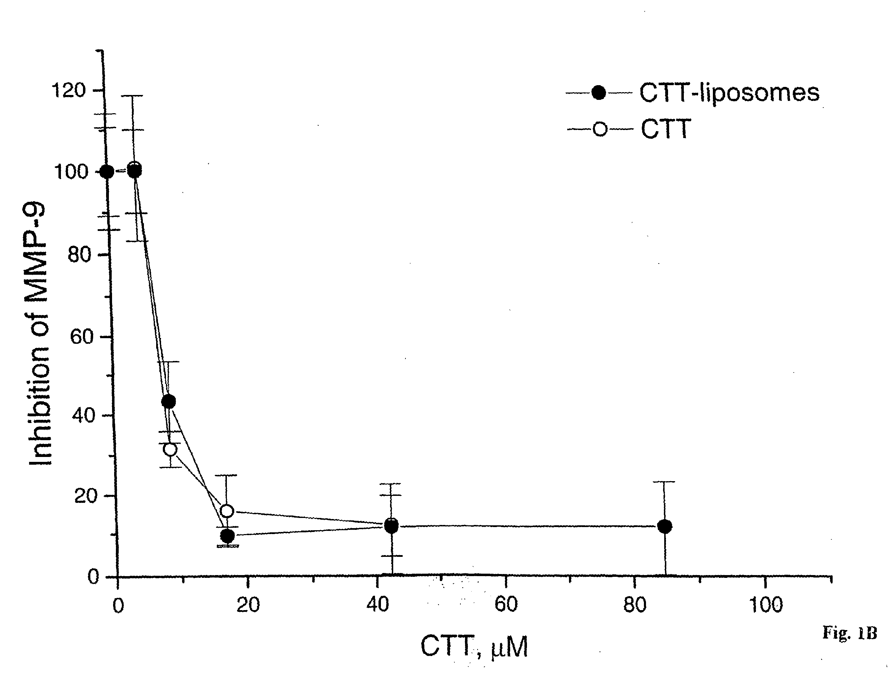

**Figure 1B.** Inhibition of MMP-9 by free CTT (o) and bound to liposomes (●) measured using the fluorogenic substrate as described in Materials and Methods. Total concentration of the phospholipid was 200 µM POPC/POPE (80/20 mol/mol). Data points represent mean values from triplicate experiments with bars illustrating standard deviation.

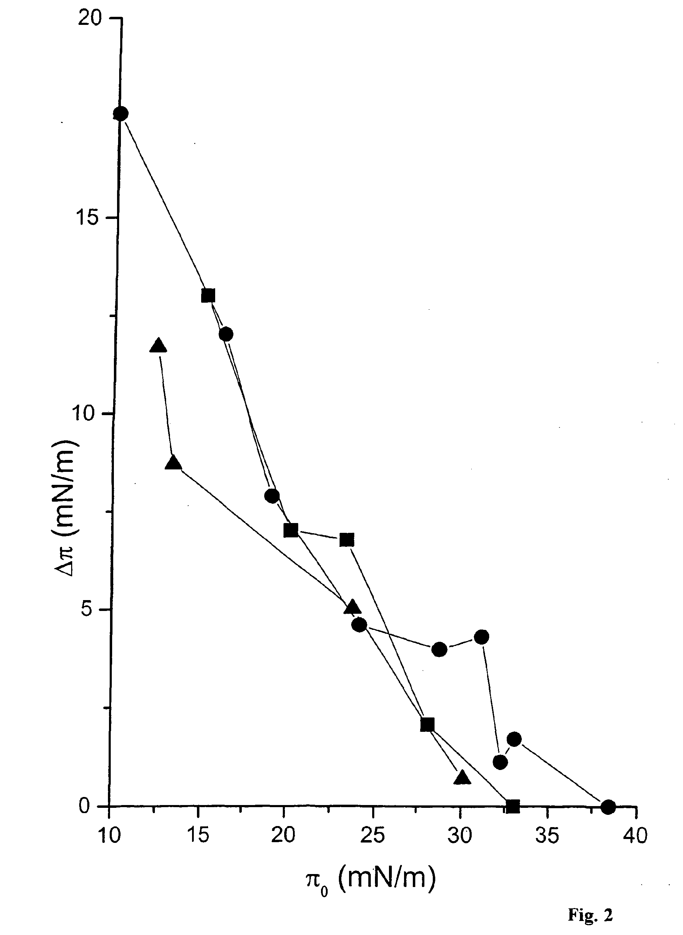

**Figure 2.** Penetration of CTT (●), CLP (■), and STT (▲) into an eggPC monolayer, evident as an increase in surface pressure ($\Delta\pi$) after the addition of the indicated peptide into the aqueous subphase. Data are shown as a function of the initial surface pressure ($\pi_0$).

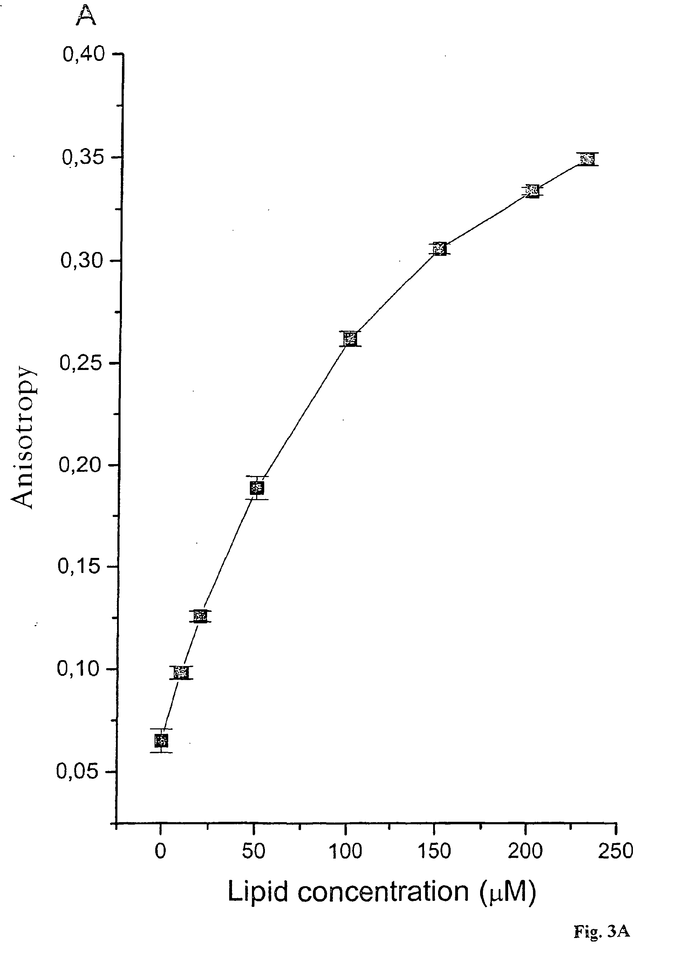

**Figure 3A.** Anisotropy (r) for the Trp residue of CTT as a function of POPC/POPE (80/20 mol/mol) concentration. The data points represent the average from five measurements with error bars giving the standard deviations. To improve signal-to-noise ratio the signal was averaged for 15 sec. Concentration of CTT was 5 µM in PBS and temperature was 37°C.

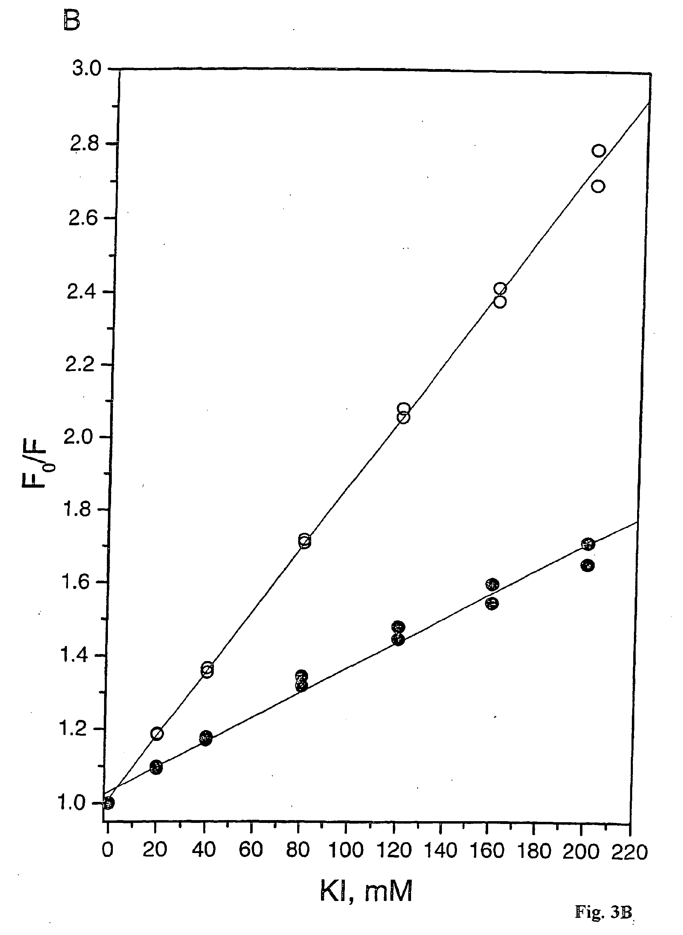

**Figure 3B.** Trp emission intensity for free CTT peptide (o) and its liposome complex (•) was measured using I⁻ as a water soluble collisional quencher. The data points represent two separate measurements.

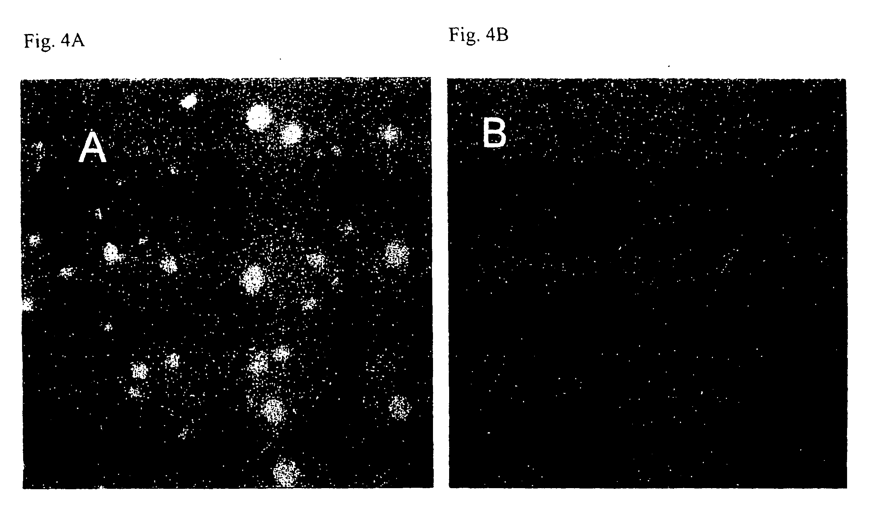

**Figure 4A to 4F.** Fluorescence microscopy images of rhodamine B intake by U937, CHO, and HT1080 cells incubated with PC/PE (80/20, molar ratio) liposomes with the encapsulated fluorescent marker and with the targeting peptide CTT, as indicated. *Panels A and B* show U 937 cells, which have been incubated with liposomes with and without CTT, respectively. *Panels C and D* illustrate the same experiment for HT 1080 cells and *panels E and F* for CHO cells (exposure time prolonged ten fold).

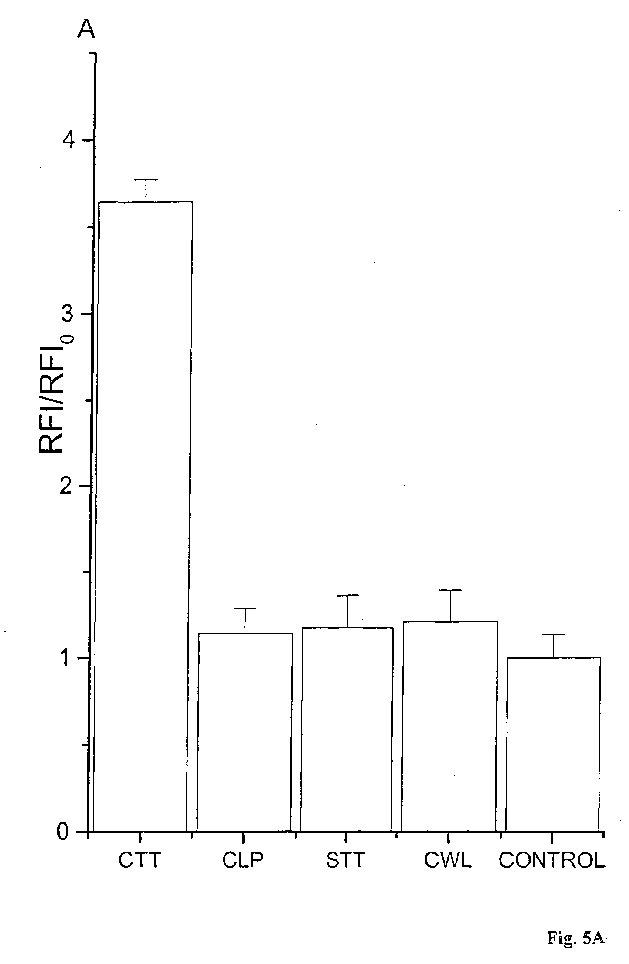

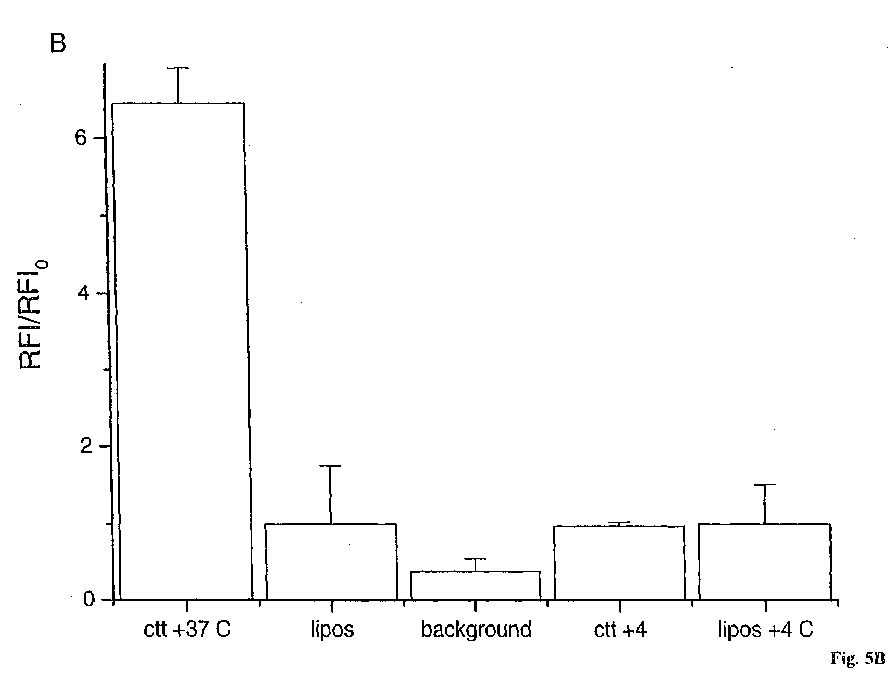

**Figure 5A.** Effects of the indicated peptides on the uptake of rhodamine B containing liposomes (PC/PE, 80/20 molar ratio) by U937 cells. Total lipid, rhodamine B, and peptide concentrations were 200 µM, 2 µM, and 100µM, respectively. The data are normalized by comparing rhodamine fluorescence of the cells incubated with liposomes with the added peptide (I) to cells incubated with liposomes without the peptides ($I_0$ = control). Error bars represent standard deviation (n=3).

**Figure 5B.** CTT was added to liposomes encapsulating the soluble fluorescent marker rhodamine B. Uptake of this fluorophore by HT1080 cells was determined following a 30 min incubation with liposomes at 37°C or 4°C. The data points are means $\pm$ SD from triplicate wells.

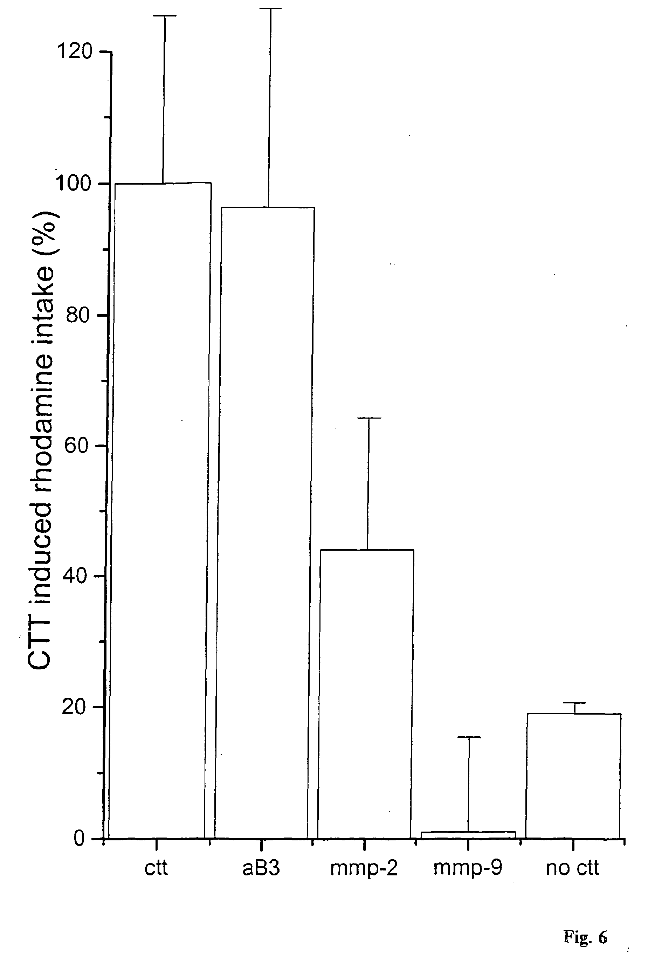

**Figure 6.** Effects of the indicated antibodies on the uptake of rhodamine B containing liposomes (PC/PE, 80/20 molar ratio) by HT 1080 cells. Results are shown as the percentage of rhodamine fluorescence of the cells incubated with liposomes with the added CTT peptide without antibodies in the medium taken as 100%. Antibodies used are anti-MMP-2, anti-MMP-9 and anti-cytosolic domain of integrin $\beta3$ which was used as a control antibody. Final concentrations of the indicated peptide, Adriamycin, antibodies, and liposomes (expressed as total phospholipid) were 85 $\mu$M, 0.2 mM, 20 $\mu$g/ml, and 0.4 $\mu$M, respectively. Error bars represent standard deviation for four separate experiments.

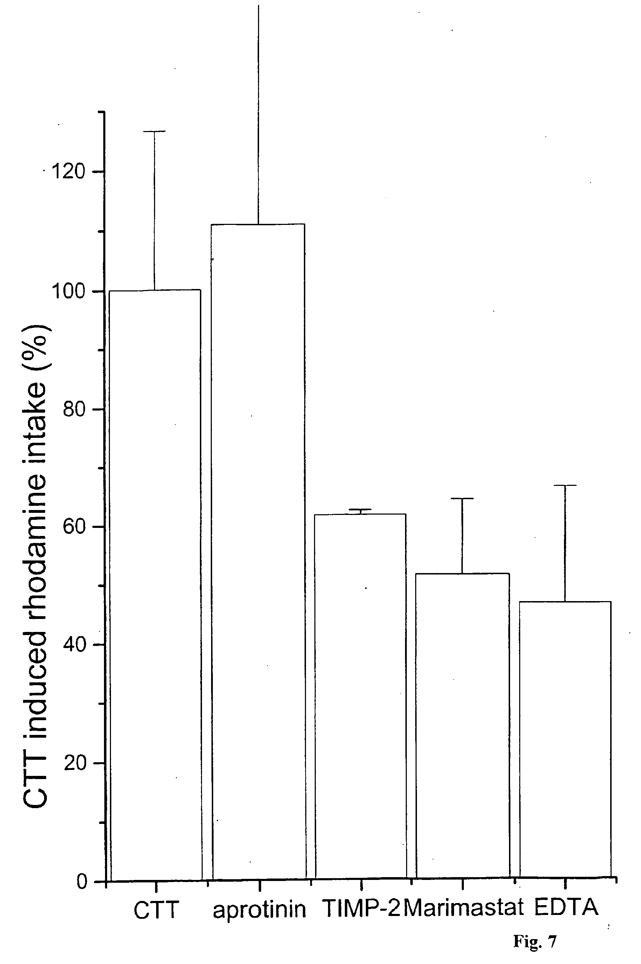

**Figure 7.** Effects of the indicated gelatinase inhibitors on the uptake of rhodamine B containing liposomes (PC/PE, 80/20 molar ratio) by HT 1080 cells. Results are shown as the percentage of rhodamine fluorescence of the cells incubated with liposomes with the added CTT peptide without the antibodies in the medium taken as 100%. The inhibitor used here is TIMP-2 (10 $\mu$g/ml). Marimastat (50 $\mu$M) is a MMP family inhibiting synthetic drug, EDTA is a $Zn^{2+}$ gelator (500 $\mu$M) and aprotinin (1 $\mu$g/ml) is a serine protease inhibitor. Final concentrations of the indicated peptide, rhodamine, and liposomes (expressed as total phospholipid) were 85 $\mu$M, 0.2 mM, and 0.4 $\mu$M, respectively. Error bars represent standard deviation for four separate experiments.

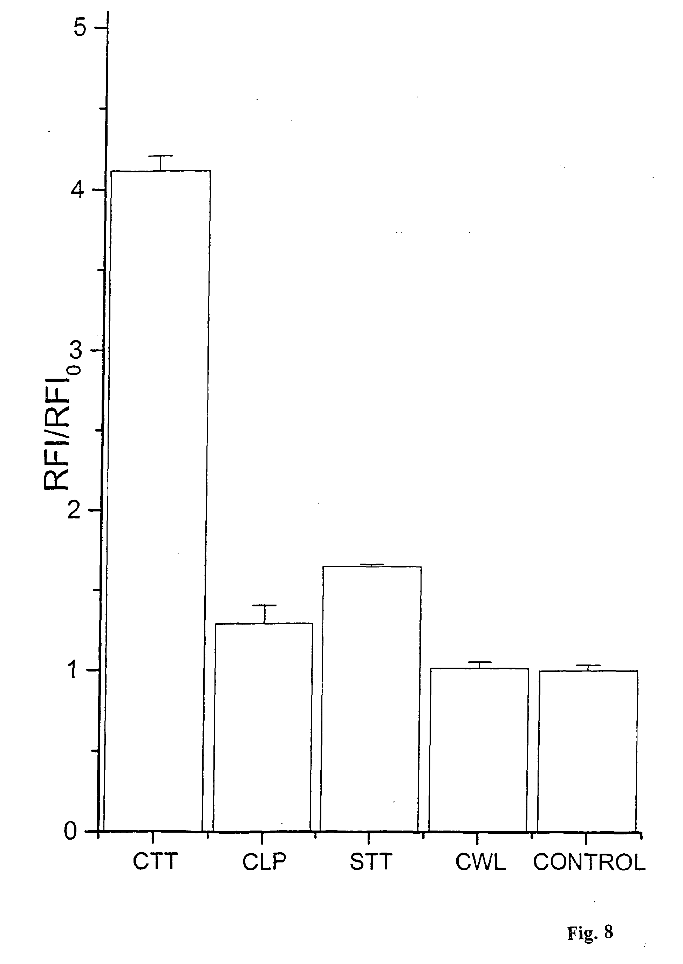

**Figure 8.** Comparison of the effects of CTT, CLP, STT, and CWL on U937 cell killing induced by Adriamycin-containing liposomes and assessed by EthD-1 fluorescence. Cells were incubated with Adriamycin (200 ng/ml) encapsulated in liposomes (200 $\mu$M total phospholipid, PC/PE = 80/20, molar ratio) and the indicated peptides. Final concentrations of the indicated peptide, Adriamycin, and liposomes (expressed as phospholipid) were 85 $\mu$M, 0.2 mM, and 0.4 $\mu$M, respectively. Results are expressed as $RFI/RFI_0$, where $RFI_0$ is the value for cells incubated with Adriamycin-containing liposomes without the peptides. Concentration of the peptides was 85 $\mu$M. Bars indicate standard deviations (n=3).

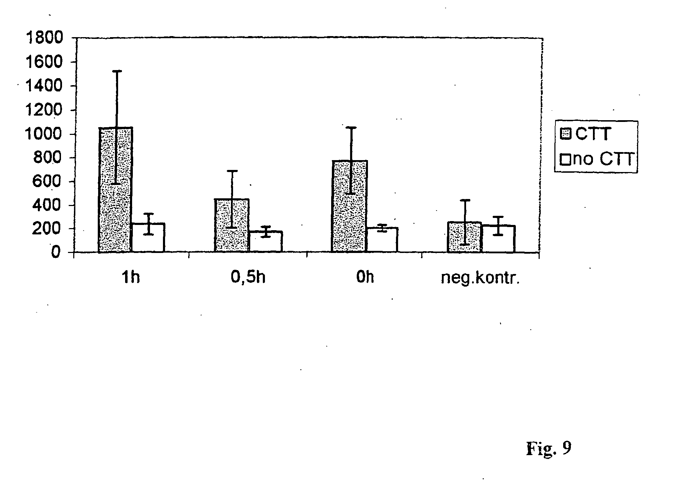

**Figure 9.** CTT-liposome intake correlates with addition of phorbol ester in short induction times, which is known to stimulate gelatinase expression, and that way gelatinases, on the cell surface. When induction time prolongs, gelatinases are located also in the medium. The amount of free gelatinase in the medium rises, whereby liposome intake is inhibited.

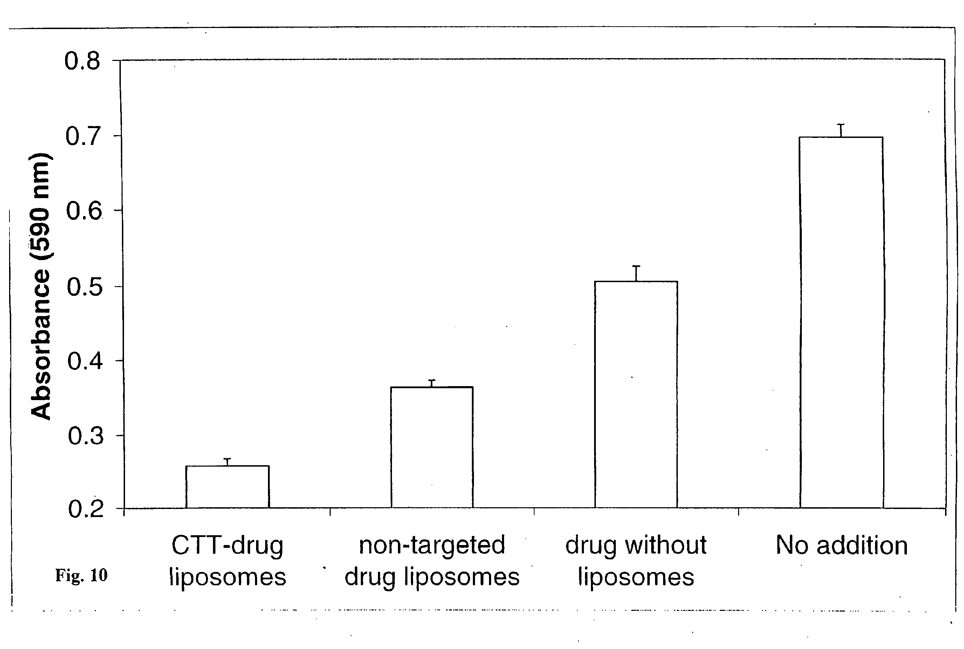

**Figure 10.** Targeting of Cytochrome c to HT1080 cells using CTT-PEG-liposomes. Cell viability is tested by MTT assay and results are given as relative fluorescence intensity at 590 nm. 'No addition' indicates cell viability without the toxic drug. 'Drug without liposomes' indicates a situation where cytochrome c is added on the cells without liposomes. 'Non-targeted drug liposomes' are pegylated liposomes without CTT, and 'CTT-drug liposomes' are pegylated liposomes where CTT peptide is attached at the far ends of PEG.

**Figure 11A to 11C.** Targeting of Tc99m-labeled CTT and/or liposomes to a tumor in nude mouse model using KS tumor model. (A) Liposomes with CTT; (B) Labeled CTT alone; (C) I-125-labeled liposomes.

## Detailed Description of the Invention

**Abbreviations:**

| | |
|---|---|
| CLP | CLPGHWGFPSC (SEQ ID NO:7) |
| CTT | CTTHWGFTLC (SEQ ID NO:6) |
| CWL | CWLTFTHGTC (SEQ ID NO:9) |
| DMEM | Dulbecco's Modified Eagle Medium |
| DMSO | dimethylsulphoxide |
| DPPE | dipalmitoylphosphatidylethanolamine |
| DPPRho | 1,2-dihexadecanoyl-*sn*-glycero-3-phosphoethanolamino-thiocarbamoyl-N-6-tetramethylrhodamine |
| ECM | extracellular matrix |

| | |
|---|---|
| EGF | epidermal growth factor |
| eggPC | egg yolk phosphatidylcholine |
| LUV | large unilamellar vesicle |
| MLV | multilamellar vesicle |
| MMP | matrix metalloproteinase |
| NBD-PE | 1-acyl-2-((7-nitro-2-1,3-benzoxadiazol-4-yl)amino)dodecanoyl-1-*sn*-glycero-3-phospho-ethanolamine |
| NHS | N-hydroxysulfosuccinimide |
| PA | phosphatidyl acid |
| PC | phosphatidylcholine |
| PE | phosphatidyl ethanolamine |
| PEG | polyethylene glycol |
| POPC | 1-palmitoyl-2-oleoyl-*sn*-glycero-3-phosphocholine |
| POPE | 1-palmitoyl-2-oleoyl-*sn*-glycero-3-phosphoethanolamine |
| RFI | relative fluorescence intensity |
| SDS | sodium dodecyl sulphate |
| STT | STTHWGFTLS (SEQ ID NO:8) |
| TIMP-2 | tissue inhibitor of metalloproteinases-2 |

**Gelatinase inhibiting peptides.** Three peptides were selected for this study (Table 1). CTT is a recently described cyclic collagenase inhibitor, which has been shown to target to tumors (Koivunen *et al.*, 1999). STT is the corresponding linear sequence of CTT in which the terminal cysteines were replaced by serines. The third peptide was CTT homolog CLP, found by sequence homology search from SWISS-PROT and EMBL databases by Blast 1.4.11 program. Since our main interest was the conserved part of CTT, our query sequence was CXXHWGFTXC (SEQ ID NO:2)(Koivunen *et al.,* 1999). Computer search revealed nine homologs, among them the sequence of CLP, which is a part of EGF-7 domain in human laminin β-1 chain (LMB1). Three of the other eight homologs were laminins from other species, one was from immunoglobulin heavy chain, and four were thiazide-sensitive sodium-chloride cotransporters from human and other species. Because this latter group had less apparent similarity to CTT, we focused instead on CLP, the CTT-resembling sequence of the laminin EGF-7 domain. Notably, all three peptides (CTT, CLP and STT) inhibited the activity of MMP-2 as assessed by casein zymography (Fig. 1). Extent of inhibition was approx. 70% at 85 μM CTT. Also CLP was a potent inhibitor of MMP-2, causing a 50% inhibition at 85 μM peptide. The linear CTT analog STT (85 μM) reduced the activity of MMP-2 by approx. 30%. Similar results were also obtained with MMP-9.

Free CTT and CTT complexed with liposomes inhibited MMP-9 also when studied by the gelatinase assay employing the fluorogenic peptide as a substrate (Fig. 1B) in keeping with the results obtained by casein zymography. The IC50 values for CTT and CTT-liposome were 8 μM in both assays. These results additionally demonstrate the MMP-9 interacting an epitope of CTT to remain available for interaction with the enzyme when bound to the lipid membrane. Complete inhibition of MMP-9 (5 nM) in this assay was obtained by 100 μM EDTA chelating the $Zn^{2+}$ required for catalysis.

**Interaction with phospholipid monolayers.** The amino acid composition of CTT readily suggests this peptide to be hydrophobic. Accordingly, it was of interest to study if CTT binds to lipids. For comparison we also investigated CLP and STT. For this purpose we used phospholipid monolayers residing on an air/buffer interface, a model biomembrane which has been widely utilized to study lipid-protein interactions and the effects of proteins on the lateral organization of lipid monolayers (Söderlund *et al.*, 1999).

Penetration of the peptide injected underneath a lipid monolayer increases the surface pressure π, and peptides which do not insert into lipid monolayers cause no changes in surface pressure. Initial surface pressure ($\pi_0$) of eggPC monolayers was varied between 10 and 40 mN/m and increments in surface pressure (Δπ) due to the addition of peptides (final concentration 200 μM) into the subphase were measured (Fig. 2). All three peptides readily penetrated into the monolayer films and the slopes of Δπ *vs*. $\pi_0$ were qualitatively similar. At initial monolayer packing pressures exceeding 38, 31, and 33 mN/m for CTT, STT, and CLP, respectively, the membrane penetration of these peptides was abolished.

**Interactions with liposomes.** The above experiments using eggPC lipid monolayers revealed CTT, CLP, and STT to bind to membranes. This was confirmed by measuring Trp fluorescence emission anisotropy for CTT in the presence of increasing concentrations of liposomes (Fig. 3A). Accordingly, in the absence of liposomes the value for r was 0.065, reflecting rapid Brownian rotational diffusion of the peptide in solution. However, increasing concentrations of liposomes caused progressively increasing r, up to 0.349 measured at 230 μM phospholipid. Approximately half-maximal effect was evident at CTT/phospholipid molar ratio close to 5:90.

Intrinsic tryptophan fluorescence allows to estimate possible changes in the microenvironment of this fluorophore upon the association of the peptide with liposomes. The value for $I_{350}/I_{330}$ measured in PBS for the Trp residue of CTT is 1.4, whereas in the presence of LUVs (0.5 mM total phospholipid) this ratio decreased to 1.00. Trp thus resides in a more hydrophobic environment in the presence of liposomes, in keeping with partitioning of CTT into lipid membrane. In addition, the quenching of Trp by $I^-$ was reduced in the presence of liposomes, and reveals that Trp in CTT is only partially exposed to the water soluble collisional quencher I⁻. The Stern-Volmer constants were 0.0087 and 0.0034 in absence and presence of liposomes, respectively.

Some lipid-binding peptides and proteins can induce fusion of lipid vesicles. In order to explore this possibility, labelled and non-labelled LUVs were mixed in the absence and presence of peptides, and lipid mixing was measured as described earlier. However, neither CTT, STT, nor CLP caused measurable changes in the fluorescence emission intensity of NBD-PE within 15 min, revealing lack of lipid mixing and thus also vesicle hemifusion and fusion (data not shown).

**Effects on cellular uptake of liposomes.** The above data show that CTT binds to phospholipids and that CTT does not cause liposome fusion. Accordingly, it was of interest to investigate the possibility that CTT could be used in liposome targeting. We first approached this by including a fluorescent lipid marker DPPRho, into liposomes as described in Materials and Methods. Subsequently, CTT was added to these liposomes, whereafter they were added to U937 leukemia cells, serving here as a model of gelatinase-expressing cells. After 5 min the cells were washed and the fluorescence in cells measured by microplate reader. CTT promoted the association of the liposomes with U937 cells and approx. 3.7-fold more of the fluorescent marker DPPRho was bound to the cells when CTT was present (data not shown).

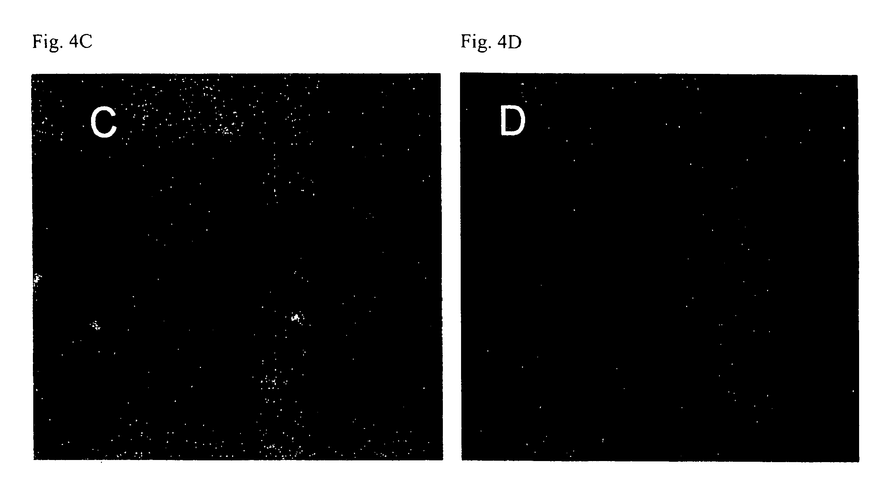

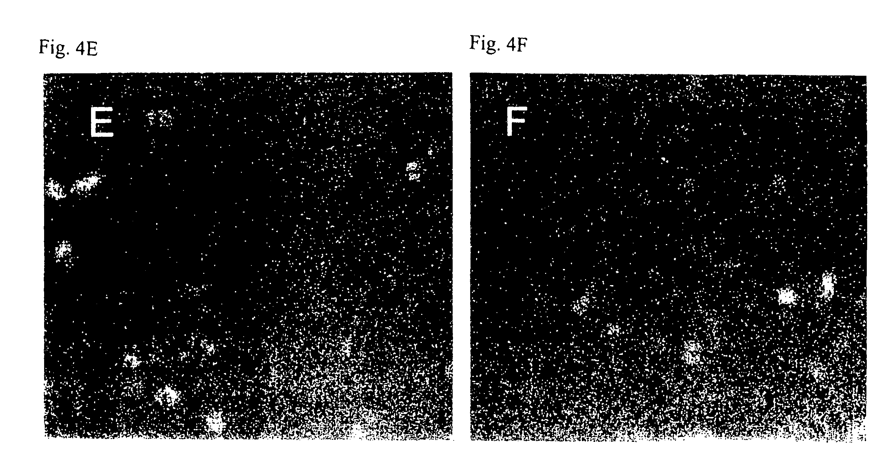

We then proceeded to study the ability of CTT to enhance the uptake by U937, CHO, NRK52E, and HT 1080 cells of the water-soluble fluorescent marker, rhodamine B encapsulated into liposomes. Same liposomes but without CTT were used as a control. Five minutes after the liposomes with CTT were added to cells, the soluble rhodamine label could be detected in cells by fluorescence microscopy. CTT enhanced rhodamine intake by U 937 and HT 1080 cells but not by CHO cells (Fig. 4). All U 937 and HT 1080 cells have fluorescence but it is distributed in an uneven manner between the cells, so some cells emit more fluorescence than others. Expression of MMP-2 as MMP-9 is essential for liposome uptake. Accordingly, Chinese hamster ovary (CHO) cells which do not express gelatinases according to our zymographic assay, showed no uptake of CTT-liposomes under conditions where an enhanced liposome uptake was evident for HT1080 and U937 cells.

Uptake of liposome-encapsulated rhodamine was quantitated by fluorescence plate-reader. The uptake of liposome-encapsulated rhodamine into U937 cells was enhanced 3.6-fold by CTT, compared to the uptake of liposomes lacking the peptide (Fig. 5A). Only minor effect was observed for STT and CLP, and the signal was too weak to be statistically significant in comparison with liposomes lacking the peptides. The scrambled cyclic peptide CWL was also inefficient in promoting the liposome uptake. Similar effect of CTT on liposome uptake was evident for human HT1080 fibrosarcoma and NRK52E rat kidney epithelial-like cells (data not shown). CTT did not promote the uptake of free rhodamine B. Enhanced liposome uptake by CTT was evident only at 37°C but not at 4°C, thus indicating active receptor-mediated endocytosis to be involved (Fig. 5B). The effect of TPA on the uptake of CTT-liposomes is biphasic and after a short exposure time (15 min) liposome intake is increased, whereas after prolonged incubation (75 min) uptake of liposomes is inhibited. This could be explained by gelatinase at low concentrations remaining bound mainly to the cell surface whereas at high concentrations the excess gelatinase locates in the extracellular space (Toth *et al.*, 1997).

**Gelatinases as targets of CTT-liposome complexes.** We further investigated if cell surface-bound gelatinases were the receptors for the CTT-containing liposomes. Cells were washed to remove soluble forms of gelatinases, and then preincubated for 30 min with MMP inhibitors or specific antibodies before the addition of liposomes. These experiments showed that the internalization of CTT-containing liposomes by HT1080 cells could be particularly efficiently prevented by antibodies to MMP-9. In particular two such MMP-9 antibodies, when used together, completely blocked the internalization of the liposome encapsulated fluorescent dye (Fig. 6). Inhibition by an antibody to MMP-2 was only partial. An antibody against cytosolic part of integrin β1 had no effect. These results indicate a specific blockade of the liposome uptake by the anti-MMP-9 and anti-MMP-2 antibodies.

Studies with a panel of proteinase inhibitors revealed that MMP inhibitors but not serine proteinase inhibitors prevented the liposome uptake. MMP inhibitors TIMP-2 and Marimastat and the cation-chelator EDTA each had a similar effect, causing an approx. 50% inhibition of liposome transfer into cells (Fig. 7). Serum trypsin inhibitors or aprotinin had no significant inhibitory effect in cells cultured in 10% fetal calf serum which internalized the liposomes.

Adriamycin, a widely used anticancer drug, has been encapsulated into liposomes with high efficiency (Gokhale *et al.*, 1996). The efficiency of CTT to promote liposome uptake and to target this therapeutic agent to tumor cells was studied in U937 culture. CTT was added to the liposome solution to a 200 μM concentration, corresponding to a CTT/phospholipid molar ratio of ~1:2. This solution was then added to U937 cells to yield final CTT and Adriamycin concentrations of 85 μM and 0.4 μM, respectively. Up to the concentrations used in this study the synthetic peptides and liposomes as such were not toxic to the cells (data not shown). Liposomes without added CTT were employed as a control and cell killing was assessed by the EthD-1 assay. After 24 h, a 4.1-fold increase in killing of the cells (p<0.001) was observed in comparison to liposomes without CTT (Fig. 8). Also gelatinase inhibiting peptides STT and CLP, but not the scrambled CWL peptide, enhanced cell killing by liposome-encapsulated adriamycin, yet to a lesser degree than CTT. Accordingly, STT and CLP increased the number of dead cells 1.7- and 1.3-fold (p<0.01), respectively.

**PEG-liposomes.** We also made PEG-liposomes, in which a PEG-lipid derivative is attached to the carboxy terminal of the CTTHWGFTLC peptide via 1-ethyl-3-(3-dimethylaminopropyl) carbodiimide hydrochloride (Grabarek and Gergely, 1990). DPPE, which has a carbamate linkage to PEG (2000), has an amine group in one of its ends. The use of PEG-lipid derivatives prolongs the *in vivo* circulation time of liposomes. It also anchors the peptide to the liposome surface, preventing the association of the peptide from the liposome in blood circulation. It can be used for targeting of tumor cells, tumor blood vessels, rheumatic arthritis lesions and other diseased tissues, where gelatinases are abundantly expressed. The molecular weights of the most commonly used PEGs are 2,000 and 5,000, but PEGs ranging from 600 to 12,000 are also used. The lipid part of the conjugate can vary from saturated or unsaturated PEs to cholesterol and ceramides with a short chain (C8), intermediate chain (C14) and long chain (C20) fatty acids. On the lipid side an infinite variety of lipid anchors from PEs to PAs, cardiolipin, cholesterol or ceramides can be used. As a functionalized group amine is used here, but all other reactive groups, which can bind peptides or modified peptides to EG-derivatives, can also be used, such as biotin, maleimide, or carboxy-NHS-ester.

CTT peptide/liposome is taken by the cells in a way that responds with adding of phorbol ester, known to stimulate gelatinase expression (Fig. 9). This shows that the gelatinase expression levels correlates with CTT-liposome intake. In our studies we have used Chinese Hamster Ovary cells, which do not express in significant level gelatinases by our gelatin zymography assays. In our results liposome targeting did not happen in the case of CHO cells. This brings us to suggest that CTT-liposome intake is cell type-dependent and correlates with gelatinase expression level of the cell type.

Liposome targeting experiments, which utilised liposomes having CTT peptide on the surface of the liposome and rhodamine inside of the liposome, revealed that liposome targeting is successful at 37 °C but not in 4 °C. This implements that active receptor-mediated endocytosis has to take place when liposomes are internalized.

CTT-liposome intake correlates with addition of phorbol ester in short induction times, which is known to stimulate gelatinase expression, and that way gelatinases, on the cell surface. When induction time prolongs, gelatinases are located also in the medium. The amount of free gelatinase in the medium rises, whereby liposome intake is inhibited (Fig. 10).

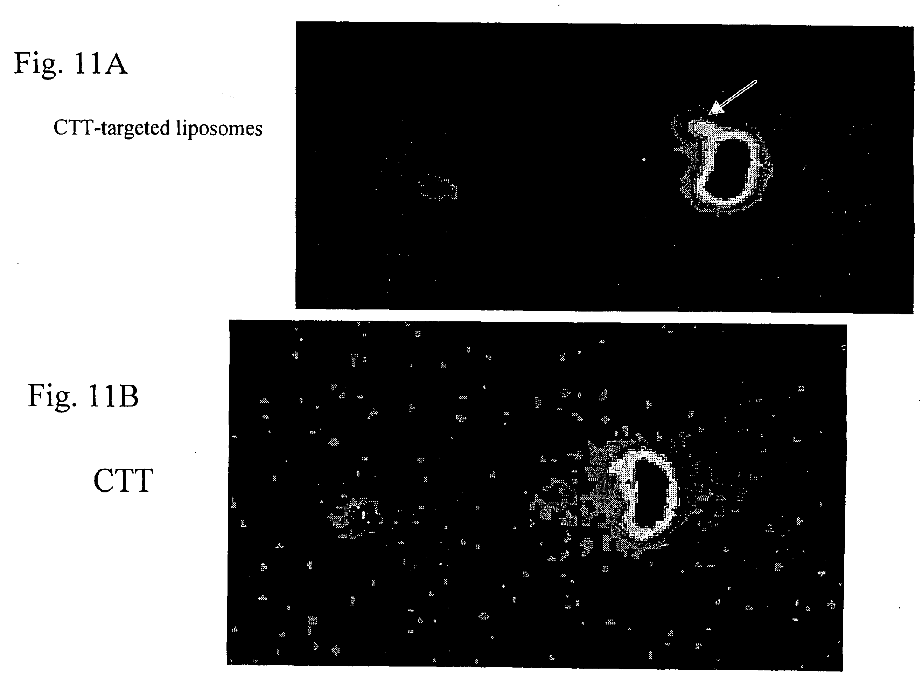

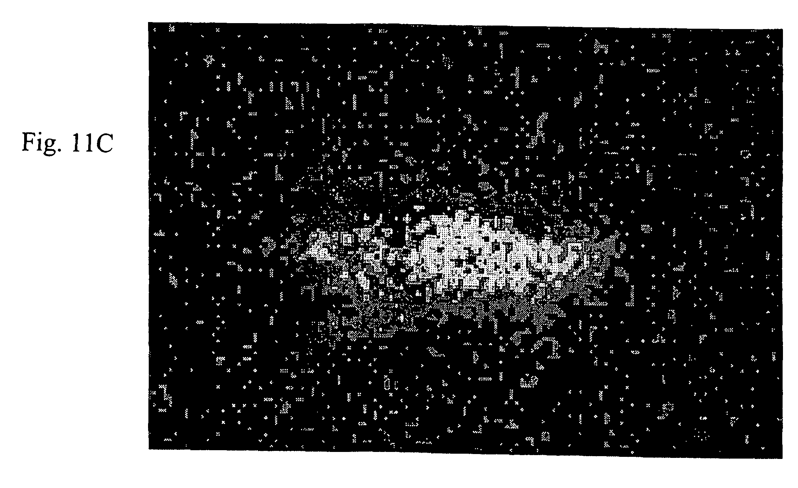

*In vivo* experiments of liposome targeting were also carried out. Figure 11A shows a gamma image of a nude mouse treated with radiolabeled CTT-peptidoliposome. CTT was labeled with Technetium-99m, which chelates between the two cysteine residues. Gamma imaging was done 30 min following the injection via the tail vein. The site of the tumor (implanted KS1767 Kaposi's sarcoma) is indicated by an arrow. Figure 11B shows a gamma image using CTT-peptide without liposomes, and Figure 11C using liposomes without CTT-peptide, labeled with radioactive Iodine-125-labeled BSA, encapsylated inside of the liposome.

**EXPER1MENTAL**

**Materials and Methods**

**Materials**. Egg yolk phosphatidylcholine (eggPC), 1-palmitoyl-2-oleoyl-*sn*-glycero-3-phosphoethanolamine, (POPE), adriamycin (doxorubicin), rhodamine B, and 0.01 M phosphate-buffered saline with 2.7 mM KCl and 0.137 M NaCl, pH 7.4 at 25°C (PBS) were purchased from Sigma and 1-acyl-2-((7-nitro-2-1,3-benzoxadiazol-4-yl)amino)-dodecanoyl-1-*sn*-glycero-3-phospho-ethanolamine (NBD-PE) and 1-palmitoyl-2-oleoyl-*sn*-glycero-3-phosphocholine (POPC) from Avanti (Birmingham, AL). The other fluorescent lipid 1,2-dihexadecanoyl-*sn*-glycero-3-phosphoethanolamino-thiocarbamoyl-N-6-tetramethylrhodamine (DPPRho), and the fluorescent probe phenanthridinium, 5,5'-[1,2-ethanediylbis(imino-3,1-propanediyl)]bis(3,8-diamino-6-phenyl)-,dichloride, dihydrochloride (EthD-1) were from Molecular Probes (Leiden, Netherlands). MMP-2 was purchased from Boehringer Mannheim GmbH (Germany). Dulbecco's Modified Eagle Medium (DMEM) and RPMI 1640 cell culture medium with Glutamax-1 were from Gibco Life Technologies (Paisley, Scotland). Phospholipid stock solutions were made in chloroform. The purity of lipids was checked by thin-layer chromatography on silicic acid coated plates (Merck, Darmstadt, Germany) using chloroform/methanol/water (65: 25:4, v/v) as a solvent. Examination of the plates after iodine staining, or when appropriate, upon fluorescence illumination revealed no impurities. Concentrations of the nonfluorescent phospholipids were determined gravimetrically using a high precision electrobalance (Cahn Instruments, Inc., Cerritos, CA, USA) and those of the fluorescent phospholipid analogs spectrophotometrically using the molar extinction coefficients $\varepsilon$= 93,000 at 540 nm for DPPRho and $\varepsilon$= 21,000 at 463 nm for NBD-PE, with methanol as a solvent. Anti-human MMP-9 and MMP-2 were kindly provided by Dr. Timo Sorsa (Department of Medical Chemistry and Periodontology, University of Helsinki, Finland). Marimastat was obtained from British Biotech. TIMP-2 and the fluorogenic substrate for MMP-2, MCA-Pro-Leu-Ala-Nva-Dpa-Ala-Arg from Calbiochem (La Jolla, CA, USA).

**Sequence homology search.** CTT (CTTHWGFTLC)(SEQ ID NO:6) homologs were searched with BLASTP 1.4.11 MP using strategy (identity matrix, word size one, and expectation value as 1000) recommended for small peptides by National Center of Bioinformation. The other parameters were kept at their default values. Our query sequence was CXXHWGFTXC (SEQ ID NO:2). Nine analogs were found, among them was a part of the EGF-7 domain in human laminin β-1 chain precursor CLPGHWGFPSC (denoted here as CLP, SEQ ID NO:7). Homologs to CLP were also searched for and were compared with the SWISS-PROT SIM program.

**Synthetic peptides.** Peptides were synthesized on an Applied Biosystems 433 A (Foster City, CA, USA) automatic synthesizer using Fmoc-chemistry. Disulfide bridges were formed in 5% acetic acid (pH 6.0) containing 20% dimethyl sulphoxide (DMSO) by incubation overnight at room temperature with continuous stirring (Domingo *et al.*, 1995). After 1:2 dilution with 0.1 % trifluoroacetic acid, peptides were loaded onto a preparative reversed phase HPLC column and eluted by an acetonitrile gradient. The identity of the peptides was verified by mass spectrometry. The peptides used in this study, with their amino acid sequences and respective abbreviations, are compiled in Table 1.

**Table 1.** Amino acid sequences of CTT, CLP, STT and CWL with the conserved residues highlighted. The molecular weights are in parentheses.

| | | |
|---|---|---|
| CTT: | Cys-Thr-Thr-**His-Trp-Gly-Phe**-Thr-Leu-**Cys** | (1168) (SEQ ID NO:6) |
| CLP: | Cys-Leu-Pro-Gly-**His-Trp-Gly-Phe**-Pro-Ser-**Cys** | (1203) (SEQ ID NO:7) |
| STT: | Ser-Thr-Thr-**His-Trp-Gly-Phe**-Thr-Leu-Ser | (1136) (SEQ ID NO:8) |
| CWL: | Cys-Trp-Leu-Thr-Phe-Thr-His-Gly-Thr-Cys | (1168) (SEQ ID NO:9) |

**Assay for gelatinases.** Inhibition of MNLP-9 and MMP-2 by the different synthetic peptides was measured using casein zymography (Halinen *et al.,* 1996). MMP-9 was purified as described (Sorsa *et al.*, 1997). Subsequently, MMP-2 (2.5 μg) or MMP-9 (2.5 μg) was run on a 10% SDS-PAGE containing two mg/ml casein. The gel was first washed in Triton X-100 containing buffer to remove SDS, and it was cut into five slices which were immersed into peptide (CTT, CLP, CWL or STT) containing solutions (85 μM). After incubation for 48 hrs at 37°C, the gels were stained with Coomassie blue, scanned, and the digested areas quantitated using image analysis (Global Lab Image 3.2, Data Translation Inc. and Acuity Imaging Inc., Marlboro, MA, USA).

The activity of MMP-9 was measured also using the fluorogenic peptide substrate MCA-Pro-Leu-Ala-Nva-Dpa-Ala-Arg (Calbiochem, San Diego, CA, USA). More specifically, 50 ng of MMP-9 was preincubated in PBS for 30 min at RT in the absence or presence of CTT or its liposome complex. Subsequently, the fluorogenic substrate was added to a final concentration of 0.1 mM and the incubation was continued at 37°C for 15 min, whereafter fluorescence intensities were measured with exitation at 340 nm and emission at 390 nm in a microtiter plate reader.

**Interaction of peptides with lipid monolayers.** Penetration of peptides into monomolecular lipid films was measured using magnetically stirred circular wells (subphase volume 400 μl). Surface pressure ($\pi$) was monitored with a Wilhelmy wire attached to a microbalance (μ TroughS, Kibron Inc., Helsinki, Finland) connected to a Pentium PC.

Lipids were spread on the air-buffer (PBS, pH 7) interface in chloroform (approx. one mg/ml) to different initial surface pressures ($\pi_0$) and were allowed to equilibrate for 15 min before the addition of the indicated peptides (4 μl, 10 mg/ml in H$_2$O and CWL in DMSO) into the subphase. The increment in $\pi$ from the initial surface pressures ($\pi_0$) after the addition of peptide was complete in approximately 20 min and the difference between $\pi_0$ and the value observed after binding of the peptide into the film was taken as $\Delta\pi$. All measurements were performed at ambient temperature (~ +24 °C). The data are represented as $\Delta\pi$ *vs.* $\pi_0$ (Brockman, 1999).

**Preparation of liposomes.** Lipid stock solutions were mixed in chloroform to obtain the desired compositions. The solvent was removed under a gentle stream of nitrogen and the lipid residue was subsequently maintained under reduced pressure for at least 2 h. Multilamellar liposomes were formed by hydrating the dry lipids at room temperature with one ml of PBS containing rhodamine B (10 μM) or adriamycin (1.8 μM) to encapsulate these compounds into liposomes, so as to yield a lipid concentration of one mM. Multilamellar liposomes were freeze/thawed five times to enhance encapsulation (Clifford *et al.*, 1990). Large unilamellar vesicles (LUVs) were obtained by extruding the MLV dispersions 19 times through a 100 nm pore size polycarbonate membrane (Nucleapore, Pleasanton, CA, USA) with a LiposoFast Pneumatic gas pressure operated small-volume homogenizer (Avestin, Ottawa, Canada). The pressure used for extrusion of vesicles through the filters was 25 psi (~170 kPa). When indicated, the peptides (2 mg/ml in PBS, except CWL in DMSO) were mixed with the LUVs to yield final lipid and peptide concentrations of 1 mM and 0.5 mg/ml, respectively.

**Preparation of PEG-liposomes** DPPE, which has a carbamate linkage to PEG (2000), has an amine group in one of its ends. PEG-lipid derivative is attached to the carboxy terminal of the CTTHWGFTLC peptide via 1-ethyl-3-(3-dimethylaminopropyl) carbodiimide hydrochloride (Grabarek and Gergely, 1990). CTT-PEG-PE is purified by gel filtration. 4% of CTT-PEG-PE is added to 16 % of POPE and 80% POPC (mol/mol) and prepared as liposomes above.

**Fluorescence spectroscopy.** The environments of the tryptophan residues of CTT, STT, CLP, and CWL in liposomes were studied by fluorescence spectroscopy. The center of Trp fluorescence peak is at ~350 nm when in water, whereas in a hydrophobic environment the emission is centered near 330 nm. Accordingly, changes in the microenvironment of Trp can be monitored by measuring $I_{350}/I_{330}$, the ratio of the emission at 350 nm to that at 330 nm (Lakowicz, 1999). Tryptophan fluorescence was recorded with a Perkin Elmer LS 50 B spectrofluorometer equipped with a magnetically stirred and thermostated cuvette compartment. All measurements were done at 37°C in 5 mM Hepes, 0.1 mM EDTA, pH 7.4 buffer. Excitation and emission bandpasses were 10 nm and 10 nm, respectively. Excitation wavelength was 295 nm, and emission spectra were recorded in the range 300 to 400 nm. Trp emission spectra of CTT, STT, CLP, and CWL were recorded both in absence of and presence of POPC/POPE (80/20, mol/mol) LUVs, yielding final lipid concentrations of 10 $\mu$M and 100 $\mu$M, as indicated.

Fluorescence polarization of the Trp residue of CTT as a function of increasing concentration of liposomes was measured using rotating polarizers in the excitation and emission beams, with a bandpass of 10 nm. The results are expressed as emission anisotropy (Lakowicz, 1999) calculated as follows:

$$r = (I_{II} - I_\perp)/(I_{II} + 2 I_\perp).$$

In this equation $I_\perp$ and $I_{II}$ represent the emission intensity perpendicular and parallel, respectively, to the excitation polarizer.

The extent of exposure of Trp to the aqueous phase was studied using I⁻ as a collisional quencher (Lakowicz, 1999). The extent of quenching was calculated by using the equation:

$$F_0/F = 1 + k[Q] = 1 + k_q \tau_0 [Q]$$

where $F_0$ and $F$ are Trp fluorescence intensities at 345 nm in the absence and presence of the quencher, Q, $\tau_0$ is Trp fluorescence lifetime in the absence of the quencher, k is the Stern-Volmer constant (obtained from the slope of the linear fit of the data). Fluorescence of Trp in CTT (5 $\mu$M) and its quenching were measured in the absence and presence of POPC/POPE (80:20, mol/mol) LUVs (final lipid concentration of 0.5 mM). The results were corrected for background caused by PBS and liposomes.

**Assay for lipid mixing.** The ability of the CTT, CLP, STT, and CWL peptides to induce liposome fusion was assessed by measuring lipid mixing, as described earlier (Struck *et al.*, 1981). In brief, NBD-PE (X=0.01) and DPPRho (X=0.01) were incorporated into liposomes (POPC/POPE, 78/20, mol/mol). Due to the spectral overlap of NBD-PE (donor) emission and DPPRho (acceptor) absorption resonance energy transfer between these dyes is highly efficient. As liposomes containing NBD-PE and DPPRho are mixed with non-labeled liposomes, fusion is observed as an increase in the emission intensity of NBD-PE due to the dilution of probes. Excitation and emission wavelengths for NBD-PE at 430 nm and 530 nm were used. All fluorescence measurements were done at 37°C.

**Assays for liposome uptake by cells.** In order to study the effects of the different peptides on the cellular uptake of liposomes, rhodamine-labelled phospholipid analog (DPPRho) was incorporated into liposomes as a tracer, to yield a composition POPC/POPE/DPPRho (80:19:1, mol/mol). The indicated peptides were mixed with LUVs and subsequently added to cells cultured in microtiter well plates. After an incubation for 5 min at 38°C liposomes not bound to the cells were removed by rinsing the cells three times with cold PBS. The relative amount of DPPRho associated with the cells was determined using Tecan Spectrafluor Plus microplate reader (Tecan, Hombrechtigon, Switzerland) with excitation at 535 nm and emission at 595 nm.

In another series of experiments the water-soluble fluorescent marker rhodamine B was encapsulated into liposomes and its uptake by cells was measured. Samples for rhodamine B uptake were prepared by mixing 60 $\mu$l of POPC/POPE (80/20, mol/mol, one mM) liposomes containing 10 $\mu$M rhodamine B to 300 $\mu$l of DMEM medium supplemented with 10% fetal bovine serum, Glutamax I, penicillin 100 U/ml and streptomycin 0.1 mg/ml. Peptides were then added to obtain a final concentration of 8.5 $\mu$M. Ten $\mu$l ($10^5$ cells) of medium containing the indicated cells were combined with the LUVs. After an incubation for 15 min at 37°C or +4°C liposomes not bound to the cells were removed from 96-well plates by rinsing three times with cold PBS. The +4°C temperature served as a control for non-endocytotic liposome uptake by cells (Oess and Hildt, 2000). The relative amount of rhodamine B associated with the cells was determined using microplate reader with excitation at 535 nm and emission at 595 nm.

We also preincubated cells with phorbol ester (TPA) (50 nM). Preincubation times varied from 15 to 75 min. Treatment of cells by phorbol ester (TPA) increases both expression and secretion of MMP-9 (Toth *et al.*, 1997). Results are presented as RFI=I/$I_0$, where RFI is relative fluorescence intensity, $I_0$ is the emission intensity for the control, 2 $\mu$M rhodamine B in LUVs without the peptides, and I is the rhodamine emission for the peptide-liposome complexes taken up by the cells. Effects of the indicated antibodies ($\alpha$MMP-9 and $\alpha$MMP-2) and gelatinase inhibiting compounds (TIMP-2, Marimastat and EDTA), on the uptake of rhodamine B containing liposomes (PC/PE, 80/20 molar ratio) by HT 1080 cells was performed as above. M-17 inhibits the binding of MMP-9 to the substrate and this inhibition is expected to be concentration dependent. It is not known if rabbit polyclonal antibody blocks human MMP-2 (Murphy *et al.*, 1994). Final concentrations of the indicated peptide, Adriamycin, antibodies and liposomes were 85 $\mu$M, 0.2 mM, 20 $\mu$g/ml, and 0.4 $\mu$M, respectively.

**Cell cultures and fluorescence microscopy.** U937 (ECACC 85011440), HT 1080 (ECACC 85111505), and CHO (ECACC 85050302) cells were cultured in RPMI or DMEM medium supplemented with 10% fetal bovine serum, Glutamax 1, penicillin (100 U/ml), and streptomycin (0.1 mg/ml). Uptake of rhodamine B containing liposomes by the cultured cells was verified by fluorescence microscopy. An inverted fluorescence microscope (Zeiss IM 35) equipped with Nikon extra long working distance objectives (20x) and (40x) were used. Samples were prepared as described for the rhodamine uptake assay, with slight modifications. Accordingly, instead of washing, U937, HT 1080, and CHO cells with the rhodamine-containing liposomes and in cell culture medium were transferred into Nunclon 48 plate wells. Excitation and emission wavelengths were selected by suitable bandpass filters (Melles-Griot) transmitting in the range 535 nm and >600 nm, respectively. Fluorescence images were viewed with a Peltier-cooled digital camera (C4742-95, Hamamatsu, Japan) connected to a computer and operated by software (Hipic 5.0) provided by the instrument manufacturer.

**Viability assays.** Peptide-liposome complexes with the encapsulated adriamycin were made as described above, yielding final concentrations of peptide, lipid, and adriamycin of 85 $\mu$M, 0.2 mM, and 0.4 $\mu$M, respectively, and were subsequently added to a ten $\mu$l ($10^5$ cells) sample of U937 cells. For the detection of dead cells EthD-1 was used. This fluorophore is membrane-impermeable and emits fluorescence only when bound to DNA (Papadopoulos *et al.*, 1994). Accordingly, EthD-1 readily enters dead cells and binds to their DNA, its fluorescence intensity correlating to the amount of dead cells. EthD-1 emission was measured using microplate reader with excitation at 495 nm and emission at 635 nm. Student's t-test was used to assess statistical significance.

*In vivo* **liposome targeting experiment.** Iodination of BSA was performed by the Iodogen 1,3,4,6-tetrachloro-3,6-diphenylglycoluril method. Briefly, 100 $\mu$l (10 mg/ml) of BSA and $^{125}$I (50 MBg) were mixed in a Iodogen-coated vial. To separate free Iodogen PD-10 gel column was used to filter the reaction products. The forming of liposomes POPC/POPE (80:20 mol/mol) and encapsylation of Iodinated BSA was made as described earlier. $^{99m}$Tc labeled CTT-peptide was made by doc. SL Karonen from Helsinki University Hospital. Injected concentrations of CTT and lipids were 8.5 to 85 $\mu$M and 0.2 mM, respectively. Injection was performed via the tail vein and the volume varied from 50 $\mu$l to 200 $\mu$l. The final plasma concentration of the peptide was 0.085 $\mu$M to 17 $\mu$M.

Gamma imaging was done 30 min following the injection. Gamma imaging was performed with a Picker Prism 1500XP single-head gamma camera connected to an Odyssey computer (Picker International, Highland Heights, OH). 25 nude NMRI mice weighting from 16 to 21 g, with Kaposi's sarcoma tumors implanted on their back.

**Sequence listing free text:**

For SEQ ID NO:1 to SEQ ID NO:5
Variable aa, Xaa in position 2 (3,4,8,9,10) can be any amino acid

**References**

Adlakha-Hutcheon, G., Bally, M.B., Shew, C.R., and Madden, T.D. Controlled destabilization of a liposomal drug delivery system enhances mitoxantrone antitumor activity. Nature Biotechnol. *17*: 775-779, 1999.

Brockman, H. Lipid monolayers: why use half a membrane to characterize protein-membrane interactions? Curr. Opin. Struct. Biol. **9**:425-427, 1999.

Brooks, P.C., Stromblad, S. Sanders, L.C., von Schalscha, T.L. Aimes, R.T. Stetler-Stevenson, W.G. Quigley, J.P., and Cheresh, D.A. Localization of matrix metalloproteinase MMP-2 to the surface of invasive cells by interaction with integrin $\alpha_v\beta_3$. Cell 85: 683-693, 1996.

Clifford, C.J., Warren, E.L. Richard, T.W., and Pfeiffer, D.R. Factors affecting solute entrapment in phospholipid vesicles prepared by the freeze-thaw extrusion method: a possible general method for improving the efficiency of entrapment. Chem. Phys. Lipids *55*: 73-83, 1990.

Domingo, G.J., Leatherbarrow, R.J., Freeman, N., Patel, S., and Weir, M. Synthesis of a mixture of cyclic peptides based on the Bowman-Birk reactive site loop to screen for serine protease inhibitors. Int. J. Peptide Protein Res.

*46*: 79-87, 1995.

Gokhale, P.C., Radhakrishnan, B., Husain, S.R., Abernethy, D.R., Sacher, R., Dritschilo, A., and Rahman, A. An improved method of encapsulation of doxorubicin in liposomes: pharmacological, toxicological and therapeutic evaluation. Br. J. Cancer *74:* 43-48, 1996.

Grabarek, Z. and Gergerly, J. Zero-length crosslinking procedure with the use of active esters. Anal. Biochem. 185, 131-135, 1990.

Halinen, S., Sorsa, T., Ding, Y., Ingman, T., Salo, T., Konttinen, Y.T., and Saari, H. Characterization of matrix metalloproteinase (MMP-8 and -9) activities in the saliva and in gingival crevicular fluid of children with Down's syndrome. J. Periodontology *67*: 748-754, 1996.

Koivunen, E., Arap, W,. Valtanen, H., Raininsalo, A., Penate Medina, O., Heikkilä, P., Kantor, C., Gahmberg, C. G., Salo, T., Konttinen, Y.T., Sorsa, T., Ruoslahti, E., and Pasqualini, R. Cancer therapy with a novel tumor-targeting gelatinase inhibitor selected by phage peptide display. Nature Biotechnol. *17*: 768-774, 1999.

Lakowicz, J.R. Principles of Fluorescence Spectroscopy. Plenum Press, New York and London, Ch 5, pp. 111-153, 1999.

Lasic, D.D., Ceh, B., Stuart, M.C., Guo, L., Frederik, P.M., and Barenholz, Y. Transmembrane gradient driven phase transitions within vesicles: lessons for drug delivery. Biochim. Biophys. Acta *1239*:145-156, 1995.

Lauhio, A., Leirisalo-Repo, M., Lähdevirta, J., Saikku, P., and Repo, H. Doubleblind, placebo-controlled study of the three month treatment with lymecyclinein reactive arthritis, with special reference to *Chlamydia* arthritis. Arthritis Rheum. *24*: 6-14, 1991.

Murphy, G., Nguyen, Q., Cockett, M., Atkinson, S., Allan, J., Knight, C., Willenbrock, F., Docherty, A. Assessment of the role of the fibronectin-like domain of gelatinase A by analysis of a deletion mutant. J. Biol. Chem. *269*: 6632-6636, 1994.

Northfelt, D.W., Martin, F.J., Working, P., Volberding, P.A., Russell, J., Newman, M., Amantea, M.A., and Kaplan, L.D. Doxorubicin encapsulated in liposomes containing surface-bound polyethylene glycol: pharmacokinetics, tumor localization, and safety in patients with AIDS-related Kaposi's sarcoma. J. Clin. Pharmacol. *36*: 55-63, 1996.

Oess, S., and Hildt, E. Novel cell permeable motif derived from the PreS2-domain of hepatitis-B virus surface antigens. Gene Therapy. **7**:750-758, 2000.

Papadopoulos, N. G., Dedoussis, G.V., Spanakos, G., Gritzapis, A.D., Baxevanis, C.N., and Papamichail, M. An improved fluorescence assay for the determination of lymphocyte-mediated cytotoxity using flow cytomethry. J. Immunol. Meth. *177*: 101, 1994.

Sears, B.D. (1984). Synthetic Phospholipid Compounds. US Patent 4,426,330.

Shapiro, S.D. Mighty mice: transgenic technology "knocks out" questions of matrix metalloproteinase function. Matrix Biol. *15*: 527-533, 1997.

Sorsa, T., Ding, Y., Salo, T., Lauhio, A., Teronen, O., Ingman, T., Ohtani, H., Andoh, N., Takeha, S., and Konttinen, Y. T. Effects of tetracyclines on neutrophil, gingival, and salivary collagenases. A functional and western-blot assessment with special reference to their cellular sources in periodontal diseases. Ann. N. Y. Acad. Sci. *732*: 112-131, 1994.

Sorsa, T., Salo, T., Koivunen, E., Tyynelä, J., Konttinen, Y.T., Bergmann, U., Tuuttila, A., Niemi, E., Teronen, O., Heikkilä, P., Tschesche, H., Leinonen, J., Osman, S., and Stenman. Activation of type IV procollagenases by human tumor-associated trypsin-2. J.Biol. Chem. *272*: 21067-21074, 1997.

Storm, G., and Crommelin, D. J. A. Liposomes: quo vadis? Pharm. Sci. & Tech. Today *1*: 19-31, 1998.

Struck, D.K., Hoekstra, D., and Pagano, R.E. Use of resonance energy transfer to monitor membrane fusion. Biochemistry *20*: 4093-4099, 1981.

Söderlund, T., Lehtonen, J.Y.A., and Kinnunen, P.K.J. Interactions of cyclosporin A with phospholipid membranes: effect of cholesterol. Mol. Pharm. *55*: 32-38, 1999.

Tardi, P.G., Boman, N.L., and Cullis, P.R. Liposomal doxorubicin. J. Drug Target. *4*:129-140, 1996.

Toth, M., Gervasi, D.C., and Fridman, R. Phorbol ester-induced cell surface association of matrix metalloproteinase-9 in human MCF10A breast epithelial cells. Cancer Res. *57*: 3159-3167, 1997.

**Claims**

1. Use of peptide compounds comprising a structure selected from the group consisting of the cyclic peptide CLPGHWGFPSC and peptides having the linear motif of S(X)yHWGFXXS, wherein X is any amino acid residue and y is an integer of 2 or 3, in a diagnostic or imaging composition for improving targeting of liposomes to tumor cells.

2. Use of peptide compounds comprising a structure selected from the group consisting of the cyclic peptide CLPGHWGFPSC and peptides having the linear motif of S(X)yHWGFXXS, wherein X is any amino acid residue and y is an integer of 2 or 3, in a diagnostic or imaging composition for enhancing the uptake of liposomes to tumor cells.

3. Use according to claim 1 or 2, wherein the peptide compound is selected from the group consisting of peptides CLPGHWGFPSC and STTHWGFTLS.

4. Use according to any one of claims 1 to 3, wherein the tumor cells are tumor cells expressing matrix metalloproteinases.

5. Use according to any one of claims 1 to 4, wherein the liposomes are carrying at least one chemotherapeutic agent.

6. Use according to claim 5, wherein the chemotherapeutic agent is adriamycin.

7. Use according to any one of claims 1 to 6, wherein the liposomes further comprise polyethylene glycol (PEG).

8. Use of a composition comprising liposomes carrying at least one chemotherapeutic agent and at least one peptide compound selected from the group consisting of the cyclic peptide CLPGHWGFPSC and peptides having the linear motif of S(X)$_y$HWGFXXS, wherein X is any amino acid residue and y is an integer of 2 or 3 for the manufacture of a medical composition to treat tumor cells of a patient.

9. A diagnostic or imaging test kit for carrying out a diagnostic method for detecting a suspected tumor in a patient, wherein the test kit comprises

   - liposomes,
   - at least one peptide compound selected from the group consisting of the cyclic peptide CLPGHWGFPSC and peptides having the linear motif of S(X)$_y$HWGFXXS, wherein X is any amino acid residue and y is an integer of 2 or 3, and
   - a detectable label.

10. A diagnostic or imaging composition, comprising

    - liposomes,
    - at least one peptide compound selected from the group consisting of the cyclic peptide CLPGHWGFPSC and peptides having the linear motif of S(X)$_y$HWGFXXS, wherein X is any amino acid residue and y is an integer of 2 or 3, and
    - a detectable label.

11. The diagnostic or imaging test kit or composition according to claim 9 or 10, wherein the peptide compound is selected from the group consisting of peptides CLPGHWGFPSC and STTHWGFTLS.

12. The diagnostic or imaging test kit or composition according to claim 9 or 10, wherein the detectable label is a radioactive label, a magnetic particle, or a fluorescent label.

**Patentansprüche**

1. Verwendung von Peptidverbindungen, umfassend eine Struktur ausgewählt aus der Gruppe, die aus dem cyclischen Peptid CLPGHWGFPSC und Peptiden besteht, die das lineare Motiv von $S(X)_y$HWGFXXS aufweisen, wobei X ein beliebiger Aminosäurerest und y eine ganze Zahl von 2 oder 3 ist, in einer diagnostischen oder bildgebenden Zusammensetzung zur Verbesserung der Zielausrichtung von Liposomen auf Tumorzellen.

2. Verwendung von Peptidverbindungen, umfassend eine Struktur ausgewählt aus der Gruppe, die aus dem cyclischen Peptid CLPGHWGFPSC und Peptiden besteht, die das lineare Motiv von $S(X)_y$HWGFXXS aufweisen, wobei X ein beliebiger Aminosäurerest und y eine ganze Zahl von 2 oder 3 ist, in einer diagnostischen oder bildgebenden Zusammensetzung zur Verbesserung der Aufnahme von Liposomen in Tumorzellen.

3. Verwendung gemäß Anspruch 1 oder 2, wobei die Peptidverbindung ausgewählt ist aus der Gruppe, die aus den Peptiden CLPGHWGFPSC und STTHWGFTLS besteht.

4. Verwendung gemäß einem der Ansprüche 1 bis 3, wobei die Tumorzellen Tumorzellen sind, die Matrixmetalloproteinasen exprimieren.

5. Verwendung gemäß einem der Ansprüche 1 bis 4, wobei die Liposomen mindestens einen chemotherapeutischen Wirkstoff tragen.

6. Verwendung gemäß Anspruch 5, wobei der chemotherapeutische Wirkstoff Adriamycin ist.

7. Verwendung gemäß einem der Ansprüche 1 bis 6, wobei die Liposomen zusätzlich Polyethylenglykol (PEG) umfassen.

8. Verwendung einer Zusammensetzung, umfassend Liposomen, die mindestens einen chemotherapeutischen Wirkstoff und mindestens eine Peptidverbindung tragen, die aus der Gruppe ausgewählt ist, die aus dem cyclischen Peptid CLPGHWGFPSC und Peptiden besteht, die das lineare Motiv von $S(X)_y$HWGFXXS aufweisen, wobei X ein beliebiger Aminosäurerest und y eine ganze Zahl von 2 oder 3 ist, für die Herstellung einer pharmazeutischen Zusammensetzung zur Behandlung von Tumorzellen eines Patienten.

9. Diagnostischer oder bildgebender Testkit für die Durchführung eines diagnostischen Verfahrens zum Nachweis eines vermuteten Tumors bei einem Patienten, wobei der Testkit umfasst:

   - Liposomen,
   - mindestens eine Peptidverbindung ausgewählt aus der Gruppe, die aus dem cyclischen Peptid CLPGHWGFPSC und Peptiden besteht, die das lineare Motiv von $S(X)_y$HWGFXXS aufweisen, wobei X ein beliebiger Aminosäurerest und y eine ganze Zahl von 2 oder 3 ist, und
   - eine nachweisbare Markierung.

10. Diagnostische oder bildgebende Zusammensetzung, umfassend

   - Liposomen,
   - mindestens eine Peptidverbindung ausgewählt aus der Gruppe, die aus dem cyclischen Peptid CLPGHWGFPSC und Peptiden besteht, die das lineare Motiv von $S(X)_y$HWGFXXS aufweisen, wobei X ein beliebiger Aminosäurerest und y eine ganze Zahl von 2 oder 3 ist, und
   - eine nachweisbare Markierung.

11. Diagnostischer oder bildgebender Testkit oder diagnostische oder bildgebende Zusammensetzung gemäß Anspruch 9 oder 10, wobei die Peptidverbindung ausgewählt ist aus der Gruppe bestehend aus den Peptiden CLPGHWGFPSC und STTHWGFTLS.

12. Diagnostischer oder bildgebender Testkit oder diagnostische oder bildgebende Zusammensetzung gemäß Anspruch 9 oder 10, wobei die nachweisbare Markierung eine radioaktive Markierung, ein magnetisches Partikel oder eine fluoreszierende Markierung ist.

**Revendications**

1. Utilisation de composés peptidiques comprenant une structure choisie parmi l'ensemble constitué par le peptide cyclique CLPGHWGFPSC et les peptides ayant le motif linéaire de $S(X)_y$HWGFXXS, où X est un reste aminoacide quelconque et y est un nombre entier ayant pour valeur 2 ou 3, dans une composition de diagnostic ou de formation d'image pour améliorer le ciblage de liposomes sur les cellules tumorales.

2. Utilisation de composés peptidiques comprenant une structure choisie parmi l'ensemble constitué par le peptide cyclique CLPGHWGFPSC et les peptides ayant le motif linéaire de $S(X)_y$HWGFXXS, où X est un reste aminoacide quelconque et y est un nombre entier ayant pour valeur 2 ou 3, dans une composition de diagnostic ou de formation d'image pour augmenter la fixation de liposomes sur les cellules tumorales.

3. Utilisation suivant la revendication 1 ou 2, dans laquelle le composé peptidique est choisi parmi l'ensemble constitué par les peptides CLPGHWGFPSC et STTHWGFTLS.

4. Utilisation suivant l'une quelconque des revendications 1 à 3, dans laquelle les cellules tumorales sont des cellules tumorales exprimant des métalloprotéinases matricielles.

5. Utilisation suivant l'une quelconque des revendications 1 à 4, dans laquelle les liposomes portent au moins un agent chimiothérapeutique.

6. Utilisation suivant la revendication 5, dans laquelle l'agent chimiothérapeutique est l'adriamycine.

7. Utilisation suivant l'une quelconque des revendications 1 à 6, dans laquelle les liposomes comprennent en outre du polyéthylèneglycol. (PEG).

8. Utilisation d'une composition comprenant des liposomes portant au moins un agent chimiothérapeutique et au moins un composé peptidique choisi parmi l'ensemble constitué par le peptide cyclique CLPGHWGFPSC et les peptides ayant le motif linéaire de $S(X)_y$HWGFXXS, où X est un reste aminoacide quelconque et y est un nombre entier ayant pour valeur 2 ou 3, pour la préparation d'une composition médicale pour le traitement de cellules tumorales d'un patient.

9. Trousse de dosage pour diagnostic ou formation d'image en vue de mettre en oeuvre une méthode de détection d'une tumeur suspectée chez un patient, ladite trousse comprenant :

   des liposomes,
   au moins un composé peptidique choisi parmi l'ensemble constitué par le peptide cyclique CLPGHWGFPSC et les peptides ayant le motif linéaire de $S(X)_y$HWGFXXS, où X est un reste aminoacide quelconque et y est un nombre entier ayant pour valeur 2 ou 3, et
   un marqueur détectable.

10. Composition de diagnostic ou de formation d'image, comprenant :

   des liposomes,
   au moins un composé peptidique choisi parmi l'ensemble constitué par le peptide cyclique CLPGHWGFPSC et les peptides ayant le motif linéaire de $S(X)_y$HWGFXXS, où X est un reste aminoacide quelconque et y est un nombre entier ayant pour valeur 2 ou 3, et
   un marqueur détectable.

11. Trousse de dosage ou composition de diagnostic ou de formation d'image selon la revendication 9 ou 10, où le composé peptidique est choisi parmi l'ensemble constitué par les peptides CLPGHWGFPSC et STTHWGFTLS.

12. Trousse de dosage ou composition de diagnostic ou de formation d'image selon la revendication 9 ou 10, où le marqueur détectable est un marqueur radioactif, une particule magnétique, ou un marqueur fluorescent.

Fig. 1A

Fig. 1B

Fig. 2

Fig. 3A

Fig. 3B

Fig. 4B

Fig. 4A

Fig. 4C

Fig. 4D

EP 1 372 694 B1

Fig. 4F

Fig. 4E

Fig. 5A

27

Fig. 5B

Fig. 6

Fig. 7

Fig. 8

Fig. 9

**Fig. 10**

Fig. 11A

CTT-targeted liposomes

Fig. 11B

CTT

EP 1 372 694 B1

Fig. 11C

SEQUENCE LISTING

<110> Licentia Oy

<120> Liposome targeting of matrix metalloproteinase inhibitors

<130> 37868

<140>
<141>

<160> 9

<170> PatentIn Ver. 2.1

<210> 1
<211> 10
<212> PRT
<213> Unknown Organism

<220>
<221> SITE
<222> (2)
<223> Variable aa, Xaa in position 2 can be any amino acid

<220>
<221> SITE
<222> (3)
<223> Variable aa, Xaa in position 3 can be any amino acid

<220>
<221> SITE
<222> (8)
<223> Variable aa, Xaa in position 8 can be any amino acid

<220>
<221> SITE
<222> (9)
<223> Variable aa, Xaa in position 9 can be any amino acid

<400> 1

```
Cys Xaa Xaa His Trp Gly Phe Xaa Xaa Cys
 1               5                    10
```

<210> 2
<211> 10
<212> PRT
<213> Unknown Organism

<220>
<221> SITE
<222> (2)
<223> Variable aa, Xaa in position 2 can be any amino acid

<220>
<221> SITE
<222> (3)
<223> Variable aa, Xaa in position 3 can be any amino acid

<220>
<221> SITE
<222> (9)
<223> Variable aa, Xaa in position 9 can be any amino acid

<400> 2

```
                Cys Xaa Xaa His Trp Gly Phe Thr Xaa Cys
                 1               5                   10
```

<210> 3
<211> 11
<212> PRT
<213> Unknown Organism

<220>
<221> SITE
<222> (2)
<223> Variable aa, Xaa in position 2 can be any amino acid

<220>
<221> SITE
<222> (3)
<223> Variable aa, Xaa in position 3 can be any amino acid

<220>
<221> SITE
<222> (4)
<223> Variable aa, Xaa in position 4 can be any amino acid

<220>
<221> SITE
<222> (9)
<223> Variable aa, Xaa in position 9 can be any amino acid

<220>
<221> SITE
<222> (10)
<223> Variable aa, Xaa in position 10 can be any amino acid

<400> 3

```
Cys Xaa Xaa Xaa His Trp Gly Phe Xaa Xaa Cys
 1               5                   10
```

<210> 4
<211> 10
<212> PRT
<213> Unknown Organism

<220>
<221> SITE
<222> (2)
<223> Variable aa, Xaa in position 2 can be any amino acid

<220>
<221> SITE
<222> (3)
<223> Variable aa, Xaa in position 3 can be any amino acid

<220>
<221> SITE
<222> (8)
<223> Variable aa, Xaa in position 8 can be any amino acid

<220>
<221> SITE
<222> (9)
<223> Variable aa, Xaa in position 9 can be any amino acid

<400> 4

```
Ser Xaa Xaa His Trp Gly Phe Xaa Xaa Ser
 1           5                   10
```

<210> 5
<211> 11
<212> PRT
<213> Unknown Organism

<220>
<221> SITE
<222> (2)
<223> Variable aa, Xaa in position 2 can be any amino acid

<220>
<221> SITE
<222> (3)
<223> Variable aa, Xaa in position 3 can be any amino acid

<220>
<221> SITE
<222> (4)
<223> Variable aa, Xaa in position 4 can be any amino acid

<220>
<221> SITE
<222> (9)

<223> Variable aa, Xaa in position 9 can be any amino acid

<220>
<221> SITE
<222> (10)
<223> Variable aa, Xaa in position 10 can be any amino acid

<400> 5

```
Ser Xaa Xaa Xaa His Trp Gly Phe Xaa Xaa Ser
 1               5                  10
```

<210> 6
<211> 10
<212> PRT
<213> Unknown Organism

<220>
<223> Description of Unknown Organism:Unknown

<400> 6

```
Cys Thr Thr His Trp Gly Phe Thr Leu Cys
 1               5                  10
```

<210> 7
<211> 11
<212> PRT
<213> Unknown Organism

<400> 7

```
Cys Leu Pro Gly His Trp Gly Phe Pro Ser Cys
 1               5                  10
```

<210> 8
<211> 10
<212> PRT
<213> Unknown Organism

<400> 8

```
Ser Thr Thr His Trp Gly Phe Thr Leu Cys
 1               5                  10
```

<210> 9
<211> 10
<212> PRT
<213> Artificial Sequence

<220>
<223> Description of Artificial Sequence:Artificial

<400> 9

```
Cys Trp Leu Thr Phe Thr His Gly Thr Cys
 1               5                   10
```